# EUROPÄISCHE PATENTSCHRIFT

(11) **EP 1 506 741 B1**
(45) Veröffentlichungstag und Bekanntmachung des Hinweises auf die Patenterteilung: **22.10.2008**
(21) Anmeldenummer: 04019095.1
(22) Anmeldetag: 11.08.2004
(51) Int. Cl.: A61B 6/00, A61B 6/12, A61B 17/22

(54) **Modular koppelbare Geräteanordnung**
Modular coupled apparatus combination
Système d'accouplement modulaire

(30) Priorität: 14.08.2003 DE 10337519
(43) Veröffentlichungstag der Anmeldung: 16.02.2005
(73) Patentinhaber: Dornier Medtech System GmbH, 82234 Wessling (DE)
(72) Erfinder: Buchbauer, Peter, 85748 Garching (DE); Grözinger, Reiner, 82239 Alling (DE); Haas, Anton, 81827 München (DE); Hermecking, Hajo, Dr., 88677 Markdorf (DE); Hofsäß, Siegfried, 82140 Olching (DE)
(74) Vertreter: Grünecker, Kinkeldey, Stockmair & Schwanhäusser Anwaltssozietät

(56) Entgegenhaltungen:
- US-A- 5 488 951
- US-A- 5 836 898
- US-A- 6 119 034

## Beschreibung

Die vorliegende Erfindung betrifft eine modular koppelbare Geräteanordnung.

Es sind Geräteanordnungen dieser Gattung bekannt, bei denen eine Lithotripsieeinrichtung und eine Röntgenvorrichtung modular koppelbar sind. Dabei werden eine profilierte Seite einer Horizontalaufnahme eines C-Bogens der Röntgenvorrichtung und ein passendes Gegenstück einer Horizontalaufnahme eines Therapiekopfes der Lithotripsieeinrichtung zueinander in Anlage gebracht. Diese Anlage muss von Hand gehalten werden, um die Horizontalaufnahmen an ihren hinteren Stirnflächen mit einem bügelartigen Riegel verbinden zu können. Die Kopplungskomponenten sind aufwendig aufgebaut und der Kopplungsvorgang ist umständlich und nimmt relativ viel Zeit in Anspruch.

Ferner sind Geräteanordnungen bekannt, bei denen eine Lithotripsieeinrichtung und eine Röntgenvorrichtung mit Hilfe von rein optischen Systemen zueinander ausgerichtet werden. Dies kann mit Ungenauigkeiten behaftet sein.

Aus der US 5,836,898 ist eine Röntgenortungseinrichtung bekannt, die einen Wagen aufweist. Dieser Wagen kann unter einen Wagen einer Therapieeinheit gefahren werden, wobei ein Mittelteil des Wagens der Röntgenvorrichtung in einen Tunnel des Wagens der Therapieeinheit geschoben wird. Hierdurch wird die Therapieeinheit angehoben, so dass ihr Wagen über dem Boden schwebt. An dem Wagen der Therapieeinheit sind Koppelstifte vorgesehen, welche in nicht dargestellte Koppelöffnungen am anderen Wagen einrasten. Die Koppelstifte dienen jedoch nicht zum Sichern der gekoppelten Stellung. Vielmehr kann hierfür eine zusätzliche, nicht dargestellte Verriegelungsvorrichtung vorgesehen sein.

Aus der WO 00/69387 A1 sind ein Inkubator für frühgeborene Säuglinge und ein Gerätewagen bekannt, welche für einen gemeinsamen Transport mit Hilfe einer Kopplungsanordnung zu einem Zug zusammenkoppelbar sind. Beim Transport müssen der Inkubator und der Gerätewagen Bodenunebenheiten, Türschwellen und dergleichen passieren. Das bedeutet, dass sie relativ zueinander beweglich sein müssen. Hierzu weist die Koppelanordnung Blattfedern und ein Scharnier auf. Die Kopplungsanordnung weist einen Kopplungsschuh und ein hierzu korrespondierendes Kopplungsjoch. An dem Kopplungsjoch ist ein vertikaler Bolzen vorgesehen. Beim Erreichen der Kopplungsendlage von Kopplungsjoch und Kopplungsschuh verrastet der Bolzen in einem Rastmechanismus, wodurch das Kopplungsjoch und der Kopplungsschuh in ihrer Kopplungsendlage gesichert werden.

Die DE 295 05 579 U1 betrifft eine Einheit aus Patientenliege und Therapiekopf. Der Therapiekopf ist mit Hilfe einer Positionieranordnung, an welcher er befestigt ist, mit einer separaten Röntgenortungseinrichtung verbindbar. Der Therapiekopf und die Positionieranordnung werden zunächst angehoben. Das Anheben erfolgt mit Hilfe eines Hubseiles, das mit der Patientenliege verbunden ist. Das Anheben der Patientenliege hebt den Therapiekopf und die Positionieranordnung an. Die Positionieranordnung weist einen Stutzen auf, welcher an seiner Unterseite eine Positionierausnehmung hat. Durch Absenken des Therapiekopfes wird der Stutzen auf einer stufenförmigen Bodenplatte abgesetzt, welche an einem Bodenrahmen der Röntgenortungseinrichtung vorgesehen ist. Ein männliches Positionierteil der Bodenplatte wird hierbei in der Positionierausnehmung aufgenommen. Anschließend wird ein Schraubbolzen in das männliche Positionierteil eingeschraubt, um die erreichte Kopplungsendlange zu sichern.

Aus der US 4,684,285 ist ein Kopplungssystem bekannt, welches als Kopplungskomponenten Keilschuhe und hierzu korrespondierende Keilausnehmungen aufweist. An den Teilen ist jeweils ein Sperrmechanismus mit Rasthaken und Bedienhebel vorgesehen. Erreichen ein Keilschuh und eine Keilausnehmung ihre Kopplungsendlage, kann der betreffende Rasthaken hinter eine Kante des Teiles schwenken und so die Rückbewegung der Kopplungskomponenten sperren.

Die US 3,808,689 beschreibt eine untere und eine obere Schiene für einen Artikulator, welcher zum Aufbau von Gebissmodellen in Dentallaboren verwendet wird. Die untere und die obere Schiene sind mit Hilfe von Keilstrukturen an einen Block koppelbar.

Die DE 79 24 606 U1betrifft eine Anhängerkupplung, in welcher eine Zugdeichsel eines LKW-Anhängers eingeführt wird. In Kopplungsendlage wird die Zugdeichsel gegen ein Herausbewegen aus der Anhängerkupplung gesichert.

Die DE 91 08 026 U1 offenbart ein Kinderwagengestell, an welchem ein Kinderwagenaufsatz befestigt werden kann. Ein Trägerelement des Kinderwagengestells weist eine Lagerungsausnehmung auf, in welcher ein vom Kinderwagenaufsatz vorstehender Bolzen bis in eine Endlage eingeführt wird. Dann wird ein Arretierungselement heruntergeschwenkt, um den Bolzen in der Endlage zu sichern. Das Arretierungselement hat eine zu seiner Drehachse konzentrisch verlaufende Arretierungsausnehmung, in welcher der in Endlage befindliche Bolzen aufgenommen wird.

Bei der DE 41 06 370 C1 ist an einem Stoßwellenkopf ein Träger vorgesehen, in welchem eine Laserquelle eingebaut ist. Der Laserstrahl wird in Teilstrahlen aufgeteilt, die beide in Richtung zu einem Röntgengerät gelenkt werden. Das Ausrichten von Stoßwellenkopf und Röntgengerät erfolgt optisch mit Hilfe der Teilstrahlen.

Die DE 40 03 350 C1 offenbart eine Röntgenvorrichtung, an der zwei Laser vorgesehen sind. Die Laserstrahlen schneiden sich im Isozentrum der Röntgenvorrichtung. An einer Therapieeinheit ist als Fokusphantom eine Kugel vorgesehen. Die Kugel wird auf optische Weise mit dem Schnittpunkt der Laserstrahlen, das heißt hierüber mit dem Isozentrum der Röntgenvorrichtung, in Übereinstimmung gebracht.

Die DE 100 32 982 A1 betrifft eine Therapieeinheit. Oben auf der Therapieeinheit sind als eine Einheit eine Laserquelle und ein optoelektronischer Sensor vorgesehen. Der ausgesandte Laserstrahl trifft auf einen Punkt am C-Bogen einer Röntgenvorrichtung und wird von dort reflektiert. Die Therapieeinheit wird solange verschoben, bis der zurückgeworfene Laserstrahl so ausgerichtet ist, dass er auf den optoelektronischen Sensor trifft.

Aus der DE 38 03 566 A1 ist eine Röntgenkabine bekannt. Eine erste Röntgenröhre befindet sich an einer Seitenwand und eine zweite Röntgenröhre an der Decke. Beide Röntgenzentralstrahlen schneiden sich. Mit Laserstrahlen wird die Relativlage einer Winkelmessskala bezüglich der Röntgenzentralstrahlen bestimmt die nicht sichtbar sind.

Der vorliegenden Erfindung liegt die Aufgabe zugrunde, eine gattungsgemäße Geräteanordnung dahingehend zu verbessern, dass auf möglichst einfache Weise ein zügiges Koppeln mit guter Ausrichtung möglich ist.

Die Aufgabe wird erfindungsgemäß gelöst mit einer modular koppelbaren Geräteanordnung mit den Merkmalen des Anspruches 1.

Mit dem Einführen des Schuhs in die Ausnehmung werden der Schuh und die Ausnehmung selbstzentrierend in Anlage zueinander gebracht. Damit erfolgt gleichzeitig mit guter Genauigkeit ein schnelles Ausrichten der Lithotripsieeinrichtung zu der Röntgenvorrichtung in der vorbestimmten Lage zueinander. Mit dem Anliegen der Keilseitenflächen an dem keilartigen Ausschnitt ist die Lage der Kopplungskomponenten gleichzeitig in Richtungen in und quer zur Einführrichtung stabilisiert. Dies trifft entsprechend auf die Lage der Geräte selbst zu. Das Verbinden der Kopplungskomponenten in ihre Kopplungsendlage erfolgt mit Hilfe der Spanneinrichtung.

Bevorzugterweise kann wenigstens eine Keilseitenfläche mindestens eine nach außen seitlich hervorragende Anlagefläche aufweisen, gegenüber der sich das Querschnittsprofil des Schuhs etwa quer zu dessen Längsrichtung wenigstens abschnittsweise verjüngt. Hierdurch ergeben sich definierte Anlageflächen zwischen der Ausnehmung und dem Schuh, wodurch der Schuh in gekoppeltem Zustand besonders passgenau in der Ausnehmung anliegt.

Vorteilhafterweise kann die Anlagefläche ballig ausgerundet ausgeführt sein. Dadurch entsteht eine etwa linien- oder kantenartige Berührung zwischen Schuh und Ausnehmung, was zu einer besonders präzisen Anlage des Schuhs in der Ausnehmung führt. Außerdem sind hierdurch vertikal schiefe Lagen von Ausnehmung und Schuh zueinander ausgleichbar, wobei der Schuh trotzdem präzise in der Ausnehmung anliegt.

Günstigerweise kann der Schuh an einem vorderen Endbereich in Längsrichtung dachartig angeschrägt ausgebildet sein. Dies ermöglicht ein leichteres und schnelleres Einfädeln des Schuhs in die Ausnehmung.

Vorzugsweise kann eine der Kopplungskomponenten wenigstens abschnittsweise aus Kunststoff ausgebildet sein, vorzugsweise aus einem PTFE-Werkstoff, und die andere Kopplungskomponente wenigstens abschnittsweise aus einem Metall bestehend, vorzugsweise aus Stahl. Bei dieser Materialpaarung ergeben sich günstige Gleiteigenschaften bei der Relativbewegung von Schuh und Ausnehmung zueinander. Darüber hinaus gewährleistet die Weichheit des PTFE-Werkstoffes ein durchgängiges, wenigstens linienartiges Anliegen des Schuhes an der Ausnehmung. Dabei werden kleinere Unebenheiten der Anlageflächen durch den PTFE-Werkstoffes ausgeglichen.

In einer weiteren Ausführungsform der Erfindung kann der Schuh in seiner Längsrichtung wenigstens bereichsweise einen sich im Winkel von mindestens etwa 17° verjüngenden Querschnitt haben, vorzugsweise im Winkel von etwa 20°. Damit ist der Schuh selbsthemmungsfrei in der Ausnehmung in Anlage bringbar.

Besonders vorteilhaft können die Kopplungskomponenten jeweils im Bodenbereich der Röntgenvorrichtung und der Lithotripsieeinrichtung vorgesehen sein. Dadurch wird im unteren Bereich der Röntgenvorrichtung und der Lithotripsieeinrichtung zur Verfügung stehender Platz zum Koppeln genutzt, wobei im oberen Bereich der Röntgenvorrichtung und der Lithotripsieeinrichtung der Platz zum Handhaben der Geräte erhalten bleibt.

Vorzugsweise können die Längsrichtung der Ausnehmung und die Längsrichtung des Schuhs etwa parallel sein zu einer Ebene eines C-Bogens der Röntgenvorrichtung und zu einer Ebene eines Haltearmes der Lithotripsieeinrichtung, wenn sich die Röntgenvorrichtung und die Lithotripsieeinrichtung in gekoppeltem Zustand jeweils in einer Arbeitsstellung befinden. Damit sind die Lithotripsieeinrichtung und die Röntgenvorrichtung besonders stabil gekoppelt, insbesondere etwa quer zu den Ebenen des C-Bogens und des Haltearmes.

Besonders bevorzugt kann an einer der Kopplungskomponenten etwa senkrecht zu deren Längsrichtung ein Haltevorsprung vorgesehen sein, und an der anderen Kopplungskomponente die Spanneinrichtung vorgesehen sein, mit welcher der Haltevorsprung arretierend in Eingriff bringbar ist. Hierdurch ist ein Spannen und somit sicheres Fixieren der Kopplungskomponenten in gekoppeltem Zustand möglich.

Günstigerweise kann die Spanneinrichtung einen Exzentermechanismus mit einer Führungsausnehmung aufweisen, in welcher der Haltevorsprung führbar ist. Eine Relativbewegung der Führungsausnehmung und des Haltevorsprunges zueinander wird in eine vorbestimmte Spannbewegung der Kopplungskomponenten zueinander übersetzt.

Bevorzugterweise kann der Vorsprung eine Rolle aufweisen. Hierdurch ist der Vorsprung leichtgängig in der Führungsausnehmung bewegbar.

Vorzugsweise kann die Führungsausnehmung etwa quer zu ihrer Erstreckung eine Rasteinbuchtung aufweisen, in welcher der Haltevorsprung in Schließstellung wenigstens abschnittsweise arrretierend aufnehmbar ist. Dies hemmt in der Schließstellung ein Bewegen des Haltevorsprunges der Richtung der Erstreckung der Führungsausnehmung.

Bei einer weiteren bevorzugten Ausführungsform der Erfindung kann die Spanneinrichtung eine Rückstellfeder aufweisen, welche in Richtung einer Öffnungsstellung der Spanneinrichtung rückstellend wirkt, in welcher der Haltevorsprung in die Führungsausnehmung einführbar ist. Hiermit wird die Spanneinrichtung in eine koppelbereite Position gedrängt, wenn sie gerade nicht mit der anderen Kopplungskomponente verbunden ist.

Vorteilhafterweise kann die Rückstellfeder in einer Rückstellfederkammer der Spanneinrichtung vorgesehen sein. Damit ist die Rückstellfeder in der Spanneinrichtung integriert ausgebildet.

Bevorzugt kann wenigstens eine der Kopplungskomponenten zwischen einer Kopplungsstellung und einer Parkstellung klappbar sein. Dies erlaubt bei Nichtgebrauch der Kopplungskomponente und/oder Transport des betreffenden Gerätes eine platzgünstige Position der Kopplungskomponente.

Günstigerweise kann die Klapprichtung etwa quer zu der Längsrichtung der Ausnehmung und des Schuhs sein, in der gekoppelt wird. Bei dieser Klapprichtung bleibt die durch das Koppeln erreichte Genauigkeit der Position der Geräte zueinander erhalten.

Vorzugsweise können an einem Haltearm der Lithotripsieeinrichtung, welcher in der Ebene des C-Bogens der Röntgenvorrichtung angeordnet ist, zwei Laserpointer vorgesehen sein, die sich in einer Ausrichtung befinden, in der ihre Laserstrahlen etwa in dieser Ebene auf den C-Bogen ausgerichtet verlaufen. Damit treffen die Laserstrahlen auf einen Innenbereich des C-Bogens, wenn der Haltearm in der Ebene des C-Bogens angeordnet ist, und die Lage des Haltearms zu dem C-Bogen kann bestimmt und gegebenenfalls später besser zueinander ausgerichtet werden.

Besonders bevorzugt kann ein erster Laserpointer an einem Horizontaldrehgelenkteil des Haltearmes an einer der Abzweigungsstelle des Haltearmes von dem Gelenk etwa gegenüberliegenden Seite des Horizontaldrehgelenkteiles angeordnet sein. Bei einem Bewegen des Haltearmes bewegt sich der Laserpointer entsprechend mit. Beispielsweise kann beim Wechseln von einer Übertischposition in eine Untertischposition der Therapieeinrichtung der Laserpointer in unterschiedliche Richtungen deuten und entsprechend zum Ausrichten bezüglich des C-Bogens benutzt werden.

Besonders vorteilhaft kann ein zweiter Laserpointer an einem therapiekopfseitigen Teil des Haltearmes an einer dem Therapiekopf etwa gegenüberliegenden Seite vorgesehen sein. Bei einem Bewegen des Haltearmes bewegt sich der Laserpointer entsprechend mit. Geht man beispielsweise von einer Übertischposition in eine Untertischposition der Therapieeinrichtung, kann der Laserpointer in unterschiedliche Richtungen deuten und entsprechend zum Ausrichten bezüglich des C-Bogens benutzt werden.

In einer Weiterbildung der Erfindung kann ein Strahl des zweiten Laserpointers entlang einer schräg im Raum verlaufenden Achse verlaufen, um welche der Therapiekopf gegenüber dem ihn tragenden Haltearm schwenkbar ist. Mit diesem Strahl ist die Lage der schräg im Raum verlaufenden Achse bestimmbar.

Günstigerweise können in einem gekoppelten Zustand der Röntgenvorrichtung und der Lithotripsieeinrichtung, in welchem die Ebene des Haltearmes und die Ebene des C-Bogens annähernd zusammenfallen, die beiden Laserpointer die Innenseite einer jeweils anderen Hälfte des C-Bogens anstrahlen. Dies ist für ein Bestimmen und Justieren der Lage des Haltearmes gegenüber dem C-Bogen nutzbar.

Eine Ausführungsform der vorliegenden Erfindung ist in der Zeichnung dargestellt. Es zeigen:
- Figur 1: eine perspektivische Ansicht auf die vordere, die rechte und die obere Seite einer erfindungsgemäßen modular koppelbaren Geräteanordnung mit Lithotripsieeinrichtung und Röntgenvorrichtung,
- Figur 2: eine Ansicht auf die rechte Seite der Geräteanordnung aus Figur 1,
- Figur 3: eine Ansicht auf die vordere Seite der Geräteanordnung aus Figur 1.
- Figur 4: eine ausschnittsweise perspektivische Ansicht auf Kopplungskomponenten der Geräteanordnung in gekoppeltem Zustand,
- Figur 5: eine ausschnittsweise perspektivische Ansicht auf die Kopplungskomponenten in ungekoppeltem Zustand,
- Figur 6: eine weitere abschnittsweise perspektivische Ansicht auf die Kopplungskomponenten in ungekoppeltem Zustand,
- Figur 7: eine perspektivische Ansicht auf die vordere, die rechte und die obere Seite einer zweiten Kopplungskomponente,
- Figur 8: eine ausschnittsweise Ansicht auf die untere Seite einer Spannvorrichtung der zweiten Kopplungskomponente aus Figur 7, und
- Figur 9: eine Schnittdarstellung des in Figur 8 dargestellten Abschnittes der Spannvorrichtung in einem Schnitt gemäß einer Linie IX-IX in Figur 8.

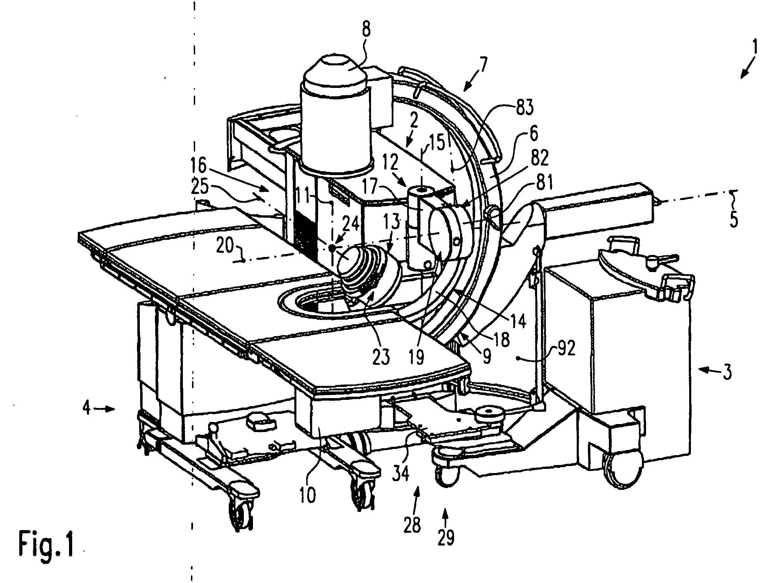

In Figur 1 ist eine perspektivische Ansicht auf die vordere, die rechte und die obere Seite einer erfindungsgemäßen modular koppelbaren Geräteanordnung 1 mit einer Lithotripsieeinrichtung 2, einer Röntgenvorrichtung 3 und einer Patientenliege 4.

Im Folgenden beziehen sich Angaben wie "vertikal" und "horizontal" oder "Bodenbereich" auf einen Boden 30, auf dem sich die Geräteanordnung 1 befindet.

Die Röntgenvorrichtung 3 weist einen um eine etwa horizontale Achse 5 schwenkbar gelagerten C-Bogen 6 auf, der an einer ersten Hälfte 7 eine Röntgenquelle 8 trägt und an einer zweiten Hälfte 9 einen Bildverstärker 10. Von der Röntgenquelle 8 zu dem Bildverstärker 10 verläuft beim Betrieb der Röntgenvorrichtung 3 ein Hauptröntgenstrahl 11, welcher einen zentralen Teil des Wirkungsbereiches der Röntgenvorrichtung 3 repräsentiert. Der Hauptröntgenstrahl 11 schneidet die etwa horizontale Achse 5 des C-Bogens 6.

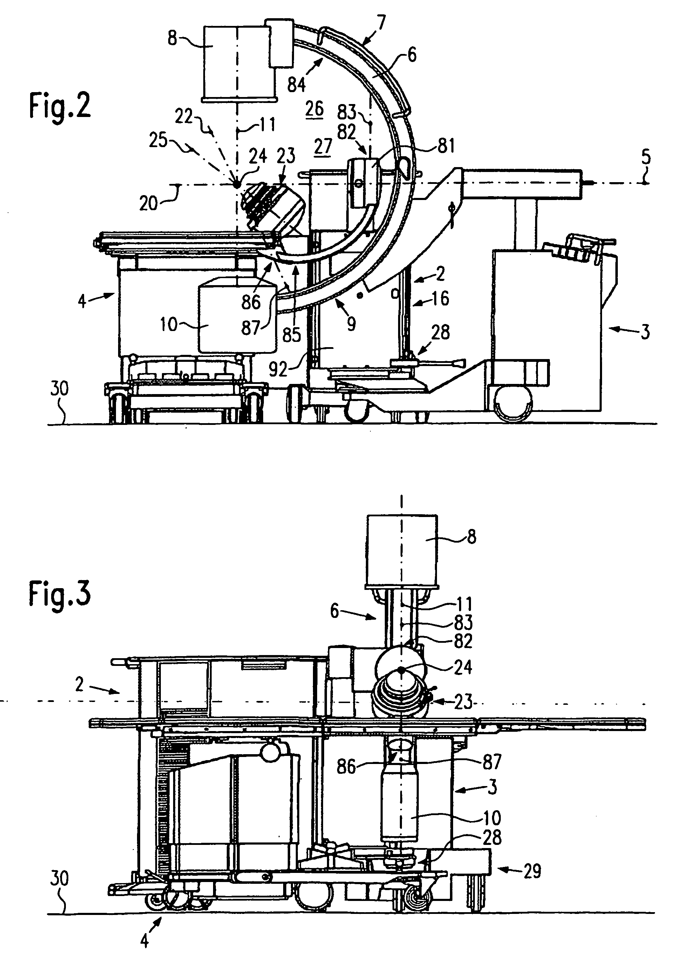

Die Lithotripsieeinrichtung 2 weist ein Vertikalgelenk 12 auf, mit welchem eine einen Therapiekopf 13 tragende Traganordnung 14 um eine etwa vertikale Achse 15 gegenüber einer Basiseinheit 16 der Lithotripsieeinrichtung 2 schwenkbar ist. Die Traganordnung 14 weist einen Horizontalarm 17 und einen C-bogenförmigen Haltearm 18 auf. Gegenüber dem mit dem Vertikalgelenk 12 verbundenen Horizontalarm 17 ist der Haltearm 18 mit Hilfe eines Horizontaldrehgelenkes 19 um eine etwa horizontale Achse 20 gegenüber der Basiseinheit 16 der Lithotripsieeinrichtung 2 schwenkbar. Die horizontale Achse 20 verläuft etwa quer zu einer Längsrichtung des Horizontalarmes 17. An dem Haltearm 18 ist über ein Schräggelenk 21 um eine schräg im Raum verlaufende Achse 22 schwenkbar der Therapiekopf 13 angeordnet, wie insbesondere Figur 2 zu entnehmen ist. Die schräg im Raum verlaufende Achse 22 schneidet die etwa horizontale Achse 20 in einem Schnittpunkt 24.

In dem Therapiekopf 13 werden Stoßwellen erzeugt und mittels akustischer Linseneinrichtungen in einem Fokus gebündelt. Der Fokus liegt auf einer Hauptwirkungslinie 25 des Therapiekopfes 13 und fällt mit dem Schnittpunkt 24 der schräg im Raum verlaufenden Achse 22 des Schräggelenkes 21 und der horizontalen Achse 20 des Horizontaldrehgelenkes 19 zusammen. Der Schnittpunkt 24 stellt gleichzeitig ein Isozentrum dar, um welches der Therapiekopf 13 um die schräg im Raum verlaufende Achse 22 und die horizontale Achse 20 des Horizontaldrehgelenkes 19 bewegbar ist.

Wie in den Figuren 1 bis 3 dargestellt, befinden sich die Traganordnung 14 und die Röntgenvorrichtung 3 in einer Arbeitsstellung relativ zueinander. Hierbei befindet sich der Haltearm 18 innerhalb des Bereiches des C-Bogens 6, die horizontalen Achsen 5, 20 der Röntgenvorrichtung 3 und des Horizontaldrehgelenkes 19 der Lithotripsieeinrichtung 2 fallen zusammen und der Hauptröntgenstrahl 11 verläuft durch das Isozentrum 24. In der Arbeitsstellung befinden sich der Haltearm 18 und der C-Bogen 6 in einer isozentrischen Ausrichtung zueinander. Dabei ist bei dieser Ausführungsform der Erfindung das Vertikalgelenk 12 lösbar in einer Stellung verrastet, in welcher der Horizontalarm 17 etwa in Längsrichtung der Basiseinheit 16 der Lithotripsieeinrichtung 2 verläuft.

Der Haltearm 18 und der C-Bogen 6 befinden sich in einer Relativlage zueinander, in welcher eine durch den Hauptröntgenstrahl 11 und die horizontale Achse 5 des C-Bogens 6 aufgespannte Ebene 26 des C-Bogens 6 und eine durch die horizontale Achse 19 und die schräg im Raum verlaufende Achse 22 des Haltearmes 18 aufgespannte Ebene 27 etwa zusammen fallen.

Um den Haltearm 18 und den C-Bogen 6 in Arbeitsstellung in ihrer isozentrischen Ausrichtung zueinander stabil zu halten, sind beide mittels einer im Bodenbereich 29 vorgesehenen Koppelanordnung 28 aneinander arretiert.

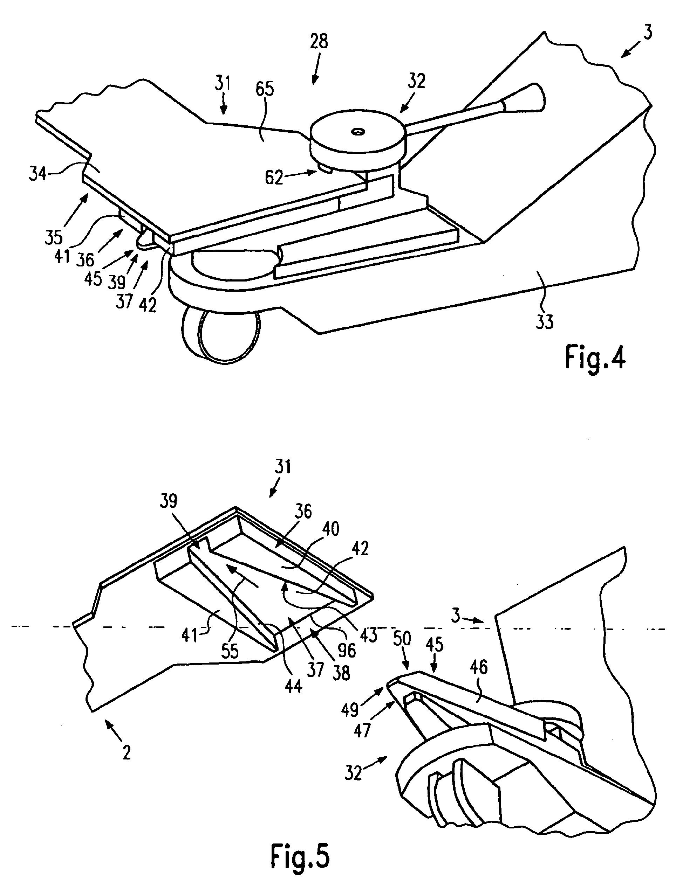

Die in den Figuren 1 bis 3 in gekoppeltem und arretiertem Zustand gezeigte Koppelanordnung 28 ist in der ausschnittsweisen Ansicht von Figur 4 detaillierter gezeigt. Die Koppelanordnung 28 weist eine erste Kopplungskomponente 31 auf, die an der Basiseinheit 16 der Lithotripsieeinrichtung 2 angeordnet ist, und eine zweite Kopplungskomponente 32, welche an einem Fahrgestell 33 der Röntgenvorrichtung 3 befestigt ist.

Die erste Kopplungskomponente 31 weist eine Halteplatte 34 auf. An einer Unterseite 35 der Halteplatte 34 ist ein Ausnehmungskörper 36 angebracht. An einer Oberseite 65 der Halteplatte 34 ist ein Haltevorsprung 62 angeordnet.

Figur 5 zeigt den Ausnehmungskörper 36 detaillierter. Der Ausnehmungskörper 36 weist eine keilförmige Ausnehmung 37 mit einer breiten Einführöffnung 38 und einer schmalen Überstandsöffnung 39 auf. Dabei verjüngt sich die Ausnehmung 37 in ihrer Längsrichtung 55 von der Einführöffnung 38 zu der Überstandsöffnung 39 hin. Die Ausnehmung 37 wird seitlich von Anlageflächen 43, 44 begrenzt, welche mit einer vertieft liegenden Oberseite 42 der Ausnehmung 37 etwa einen rechten Winkel einschließen. Die Anlageflächen 43, 44 sind Seitenflächen von Anlagebacken 40, 41 des Ausnehmungskörpers 36.

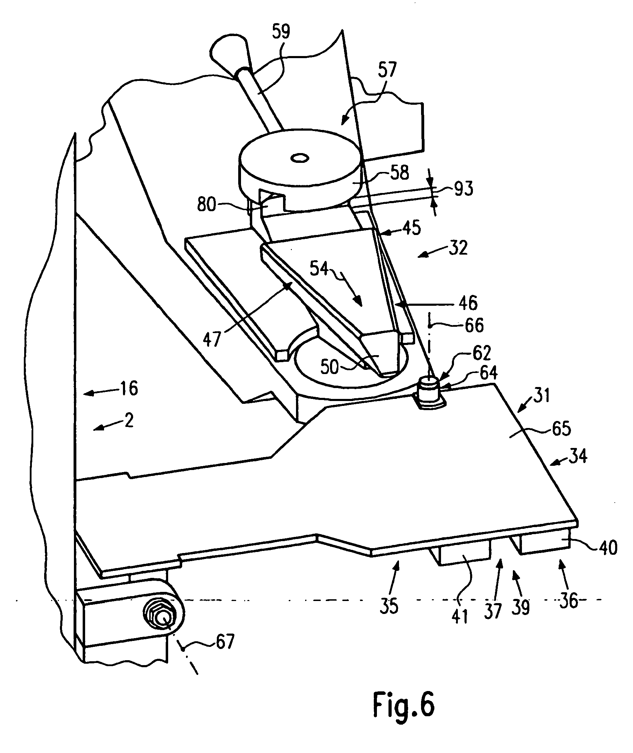

Wie Figur 6 zu entnehmen ist, ist der Haltevorsprung 62 sich etwa senkrecht von der Oberseite 65 der Halteplatte 34 erstreckend angeordnet. Der Haltevorsprung 62 befindet sich dabei in einem Bereich gegenüber dem Ausnehmungskörper 36. Der Haltevorsprung 62 weist eine Rolle 64 auf, welche um eine etwa senkrecht zu der Oberseite 65 verlaufende Rollenachse 66 drehbar ist.

Die Halteplatte 34 ist um eine etwa horizontale Klappachse 67 an der Basiseinheit 16 der Lithotripsieeinrichtung 2 schwenkbar gelagert. Die Klappachse 67 verläuft etwa parallel zu der Längsrichtung 55 der Ausnehmung 37, wodurch sich eine Klapprichtung der ersten Kopplungskomponente 31 gegenüber der Lithotripsieeinrichtung 2 etwa quer zu ihrer Längsrichtung 55 ergibt.

In einer heruntergeklappten Stellung, in der die Halteplatte 34 etwa parallel zu dem Boden 30 ausgerichtet ist, befindet sich die erste Kopplungskomponente 31 in einer Koppelstellung. In einer an die Basiseinheit 16 herangeklappten Stellung befindet sich die erste Kopplungskomponente 31 in einer Parkstellung. Sowohl in der Koppel- als auch in der Parkstellung der ersten Kopplungskomponente 31 ist eine in Figur 1 geöffnet gezeigte Fronttür 92 der Basiseinheit 16 öffen- und schließbar. Befindet sich die erste Kopplungskomponente 31 in ihrer Transportposition, wird sie bei geschlossener Fronttür 92 im Inneren der Basiseinheit 16 verborgen.

Der Ausnehmungskörper ist aus einem Kunststoff, vorzugsweise aus einem PTFE-Werkstoff hergestellt.

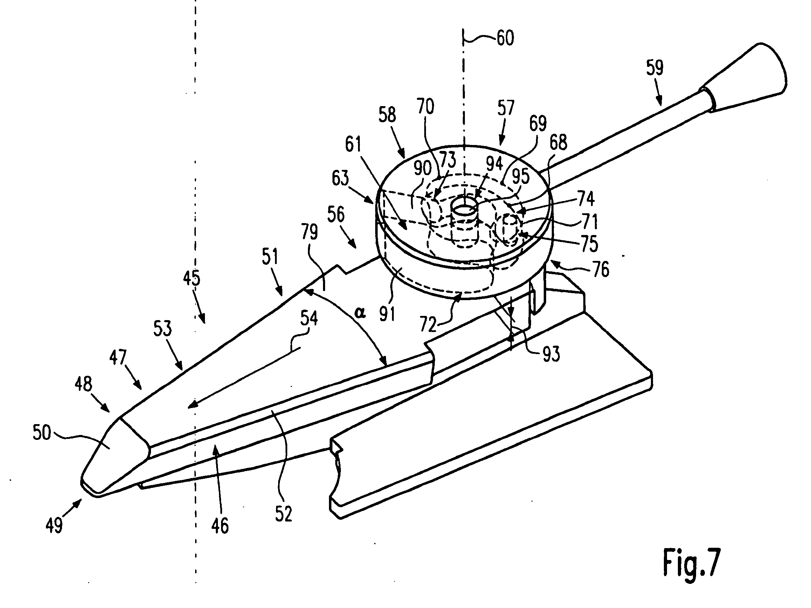

Die zweite Kopplungskomponente 32 weist einen Schuh 45 auf. Wie Figur 7 zu entnehmen ist, hat der Schuh 45 einen Keilabschnitt 51, in welchem der Schuh 45 in seiner Längsrichtung 54 keilartig ausgebildet ist. Die Längsrichtung 54 des Schuhs 45 fällt mit einer Einführrichtung des Schuhs 45 in die Ausnehmung 37 zusammen. Im Keilabschnitt 51 weist der Schuh 45 Keilseitenflächen 46, 47 auf. Die Keilseitenflächen 46, 47 weisen jeweils eine nach außen seitlich hervorragende, ballig ausgerundet ausgeführte Anlagefläche 52, 53 auf. Gegenüber den Anlageflächen 52, 53 verjüngt sich das Querschnittsprofil des Keilabschnittes 51 quer zu der Längsrichtung 54 des Schuhs 45.

An einem vorderen Endbereich 48 des Keilabschnittes 51 weist der Schuh 45 eine dachartig angeschrägte Fläche 50 auf. Die angeschrägte Fläche 50 erleichtert ein Einfädeln des Schuhs 45 in die Ausnehmung 37. Im Bereich der angeschrägten Fläche 50 hat der Schuh 45 eine gerundete Spitze 49.

Der Querschnitt des Keilabschnittes ist sich in Längsrichtung 54 des Schuhs 45 zu der Spitze 49 hin in einem Winkel α von mindestens etwa 17°, vorzugsweise etwa 20°, verjüngend ausgebildet. Hierdurch ist ein selbsthemmungsfreies Sitzen des Schuhs 45 in der Ausnehmung 37 gewährleistet.

Der Schuh ist aus einem Metall hergestellt, vorzugsweise aus Stahl.

An einem hinteren Endbereich 56 des Schuhs 45 ist ein Exzentermechanismus 57 zum Verspannen der ersten Kopplungskomponente 31 mit der zweiten Kopplungskomponente 32 vorgesehen. Der Exzentermechanismus 57 ist um einen Abstand 93 parallel von einer Oberseite 79 des Schuhs 45 beabstandet angeordnet, wie Figur 6 gut zu entnehmen ist. Dabei ist an dem hinteren Endbereich 56 des Schuhs 45 senkrecht zu der Oberseite 79 und senkrecht zu der Längsrichtung 54 des Schuhs 45 eine Anschlagfläche 80 ausgebildet.

Der Exzentermechanismus 57 weist einen zylindrischen Spannkörper 58 auf, der mit Hilfe eines Bedienhebels 59 um eine etwa vertikale Achse 60 relativ zu dem Schuh 45 bewegbar ist. Die vertikale Achse 60 verläuft durch eine Bohrung 94 des Spannkörpers 58 und durch einen darin aufgenommenen Drehstift 95.

Der Spannkörper 58 weist eine Führungsaufnehmung 61 auf, in der der Haltevorsprung 62 der ersten Kopplungskomponente 31 führbar ist. Dabei wird der Haltevorsprung 62 durch eine Einführöffnung 63 der Führungsausnehmung 61 eingeführt, wenn sich der Exzentermechanismus in einer Öffnungsstellung befindet. Die Führungsausnehmung 61 weist einen mittleren Bereich 76 auf, in dem sie sich abschnittsweise bogenförmig um die Bohrung 94 erstreckt. Zu der Einführöffnung 63 hin ist die Führungsausnehmung radial nach außen erstreckend ausgebildet.

An einem der Einführöffnung 63 abgewandtem Ende der Führungsausnehmung 61 ist eine Rasteinbuchtung 72 vorgesehen. Die Rasteinbuchtung 72 erstreckt sich radial auswärts etwa quer zu der Erstreckung der Führungsausnehmung 61. In der Rastausbuchtung 72 ist der Haltevorsprung 62 abschnittsweise aufnehmbar, wenn sich der Exzentermechanismus 57 gegenüber dem Haltevorsprung 62 in einer Schließstellung befindet.

Der Spannkörper 58 weist ferner eine Rückstellfederkammer 69 auf, in welcher eine Rückstellfeder 68 vorgesehen ist. Die Rückstellfederkammer 69 ist sich abschnittsweise um die vertikale Achse des Spannkörpers 58 bogenartig erstreckend ausgebildet. Die Rückstellfederkammer 69 weist ein erstes Ende 70 und ein zweites Ende 71 auf. Das erste Ende 70 liegt näher an der Einführöffnung 63 der Führungsausnehmung 61 und das zweite Ende 71 liegt näher an der Rasteinbuchtung 72 der Führungsausnehmung 61. Ein erstes Ende 73 der Rückstellfeder 68 liegt an dem ersten Ende 70 der Rückstellfederkammer 69 an. Ein zweites Ende 74 der Rückstellfeder 68 liegt an einem Stift 75 an, welcher sich etwa senkrecht von dem hinteren Endbereich 56 des Schuhs 45 in die Rückstellfederkammer 69 erstreckt. Die Rückstellfeder 68 wirkt in Richtung der Öffnungsstellung des Exzentermechanismus 57 rückstellend.

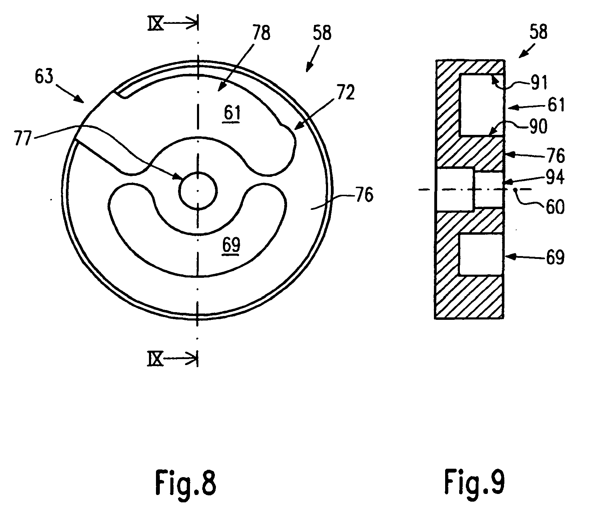

In Figur 8 ist eine Ansicht auf eine Unterseite 76 des Spannkörpers 58 gezeigt. Dieser Ansicht ist insbesondere das Profil der Führungsausnehmung 61 und der Rückstellfederkammer 69 detaillierter zu entnehmen.

Figur 9 zeigt einen Querschnitt des Spannkörpers 58 gemäß einer Linie I-I in Figur 8. In dieser Darstellung ist zu entnehmen, dass die Bohrung 94 zu der Unterseite 76 des Spannkörpers 58 sich verengend gestuft ausgebildet ist. Die Führungsausnehmung 61 und die Rückstellfederkammer 69 sind zu der Unterseite 76 des Spannkörpers 58 hin offen.

Es wird Bezug auf Figuren 1 bis 3 genommen. Der Haltearm 18 weist als einen Bestandteil des Horizontaldrehgelenkes 19 ein Horizontaldrehgelenkteil 81 auf. An dem Horizontaldrehgelenkteil 81 ist gegenüberliegend der Anbringungsstellung des Haltearmes 18 an dem Horizontaldrehgelenkteil 81 ein erster Laserpointer 82 vorgesehen. Der erste Laserpointer 82 sendet einen ersten Laserstrahl 83 aus, der in der Ebene 27 des Haltearmes 18 verläuft. Weil bei der gezeigten Stellung des C-Bogens 6 und des Haltearmes 18 zueinander deren Ebenen 26, 27 zusammenfallen, trifft der erste Laserstrahl 83 dementsprechend im Bereich der ersten Hälfte des C-Bogens 6 auf einen Innenbereich 84 des C-Bogens 6.

In der Darstellung gemäß den Figuren 1 bis 3 befindet sich dabei der Therapiekopf 23 in einer Untertischposition bezüglich der Patientenliege 4. Befindet sich der Therapiekopf 13 dagegen in einer Übertischposition bezüglich der Patientenliege 4, das heißt in einer um 180° um die horizontale Achse 20 des Horizontaldrehgelenkes 19 geschwenkten Position, trifft der erste Laserstrahl 83 im Bereich der zweiten Hälfte 9 des C-Bogens 6 auf den Innenbereich 84 des C-Bogens 6.

An einem therapiekopfseitigen Teil 85 des Haltearmes 18 ist ein zweiter Laserpointer 86 vorgesehen. Dabei ist der zweite Laserpointer 86 so ausgerichtet, dass ein von ihm erzeugter zweiter Laserstrahl 87 in der Ebene 27 des Haltearmes 18 entlang der schräg im Raum verlaufenden Achse 22 des Schräggelenkes 21 verläuft. Dementsprechend trifft der zweite Laserstrahl 87 in der in den Figuren 1 bis 3 gezeigten Position des C-Bogens 6 und des Haltearmes 18 im Bereich der zweiten Hälfte 9 des C-Bogens 6 auf die Innenseite 84 des C-Bogens 6. Dabei befindet sich der Therapiekopf 13 in der Untertischposition. Befindet sich dagegen der Therapiekopf 13 in der Übertischposition, trifft der zweite Laserstrahl 87 im Bereich der ersten Hälfte 7 des C-Bogens 6 auf die Innenseite 84 des C-Bogens 6.

Da die beiden Laserpointer 82, 86 so ausgerichtet sind, dass die von innen erzeugten Laserstrahlen 83, 87 auf die Innenseite 84 jeweils gegenüberliegender Hälften 7, 9 des C-Bogens 6 treffen, ist allein mit diesen beiden Laserpointern 82, 86 ein Bestimmen der Relativlage der Ebenen 26, 27 des Haltearmes 18 und des C-Bogens 6 zueinander möglich. Entsprechend kann mit Hilfe der Auftreffpunkte der Laserstrahlen 83, 87 auf dem Innenbereich 84 des C-Bogens 6 ein Justieren der Ebenen 26, 27 des C-Bogens 6 und des Haltearmes 18 relativ zueinander vorgenommen werden. Zusätzlich ist mit Hilfe des zweiten Laserstrahls 87 der Verlauf der schräg im Raum verlaufenden Achse 22 bestimmbar, was ebenfalls für ein Justieren nutzbar ist.

Nachfolgend wird die Funktionsweise der erfindungsgemäßen Geräteanordnung beschrieben.

Um die Lithotripsieeinrichtung 2 mit Hilfe der Koppelanordnung 28 mit der Röntgenvorrichtung 3 zu koppeln, wird zunächst die erste Kopplungskomponente 31 durch Schwenken der Halteplatte 34 um ihre horizontale Achse 67 von ihrer Parkstellung in ihre Koppelstellung geklappt. Anschließend wird die Röntgenvorrichtung 3 so bewegt, dass der Schuh 45 mit der dachartig angeschrägten Fläche 50 voran in Einführrichtung 54 in die Einführöffnung 38 der Ausnehmung 37 gelangt, vgl. Figur 6. Befindet sich dabei die Überseite 42 der Ausnehmung 37 in vertikaler Richtung zu tief gegenüber der Oberseite 79 des Schuhes 5, gleitet eine Kante 96 der Einführöffnung 38 über die dachartig angeschrägte Fläche 50 des Schuhs 45 auf die Oberseite 79 des Schuhs 45 auf. Im weiteren Verlauf des Einführens kommen die Anlageflächen 52, 53 des Schuhs 45 in Anlage mit den Seitenflächen 43, 44 der Ausnehmung 37. Durch die keilartige Ausbildung der Ausnehmung 37 und des Schuhs 45 kommt es dabei selbsttätig zu einem Ausrichten der Ausnehmung 37 und des Schuhs 45 zueinander, so dass deren Längsrichtungen 54, 55 in eine parallele Lage zueinander gebracht werden. Damit werden gleichzeitig die Lithotripsieeinrichtung 2 und die Röntgenvorrichtung 3 in ihre vorbestimmte Relativlage gebracht.

Durch die ballig ausgerundete Ausbildung der Anlageflächen 52, 53 des Schuhs 45 kommt es zu einer kanten- oder linienartigen Anlage der Anlageflächen 52, 53 an den Anlageseiten 43, 44 der Ausnehmung 37. Eventuelle Unebenheiten der Anlageseiten 43, 44 und der Anlageflächen 52, 53 relativ zueinander werden durch die Weiche des PTFE-Werkstoffes der Ausnehmung 37 ausgeglichen, in dem es zu ausgleichenden Verformungen an den Anlageseiten 43, 44 kommt. Durch die ballig ausgerundete Ausbildung der Anlageflächen 52, 53 wird ferner eine nicht parallele Lage der Oberseite 42 der Ausnehmung 37 gegenüber der Oberseite 79 des Schuhs 45 quer zu ihrer Längsrichtungen 54, 55 ausgeglichen.

Während des Einführens des Schuhs 45 in die Ausnehmung 37 befindet sich der Exzenterspannmechanismus 57 in der Öffnungsstellung. Der Haltevorsprung 62 gleitet in die Einführöffnung 63 seiner Führungsausnehmung 61 ein und die Halteplatte 34 gleitet in den Zwischenraum zwischen der Unterseite 76 des Spannkörpers 58 und der Oberseite 79 des Schuhs 45 ein. Mit Hilfe des Bedienhebels 59 wird der Spannkörper 58 im Uhrzeigersinn um seine vertikale Achse 60 gedreht, wobei der Haltevorsprung 62 relativ zu dem Spannkörper 58 in der Führungsausnehmung 61 geführt wird, bis der Haltevorsprung 62 abschnittsweise und arretierend in der Rasteinbuchtung 72 aufgenommen wird. In dieser Relativposition von Haltevorsprung 62 zu dem Spannkörper 58 befindet sich der Exzentermechanismus 57 in seiner Schließposition. In dieser Schließposition ist die Rückstellfeder 68 zwischen dem ersten Ende der Rückstellfederkammer 69 und dem Stift 75 stark komprimiert.

Mit dem Führen des Haltevorsprunges 62 in der Führungsausnehmung 61 bis zum Erreichen der Schließposition des Exzentermechanismus 57 wird zwischen den ersten und zweiten Kopplungskomponenten 31, 32 getriebeartig eine Verspannkraft aufgebaut, mit der der Schuh 45 und die Ausnehmung 37 in eine Kopplungsendlage zueinander gebracht werden. Dabei steht ein vorderer Endbereich 48 des Schuhs 45 abschnittsweise durch die Überstandsöffnung 39 der Ausnehmung 37 über, wie insbesondere Figur 4 zu entnehmen ist. Ferner ist die Halteplatte 34 mit einer im Bereich des Haltevorsprunges 62 befindlichen Seitenfläche 89 in Anlage zu der Anschlagfläche 80 der zweiten Kopplungskomponente 32 gekommen.

Beim relativen Führen des Haltevorsprunges 62 in der Führungsausnehmung 61 rollt die Rolle 64 an wenigstens einer Seitenfläche 90, 91 der Führungsausnehmung 61 ab. Dabei dreht sich die Rolle 64 um die Rollenachse 66.

Die Längsrichtung 54 des Schuhs 45 verläuft parallel zu der horizontalen Achse 5 des C-Bogens 6. Befindet sich die Traganordnung 14 in ihrer Arbeitsposition, verläuft die Längsrichtung 55 der Ausnehmung 37 parallel zu der horizontalen Achse 20 des Horizontaldrehgelenkes 19. Da in gekoppeltem Zustand die Längsrichtungen 54, 55 des Schuhs 45 und der Ausnehmung 37 parallel zueinander sind, sind auch die horizontalen Achsen 5, 20, des C-Bogens und des Horizontaldrehgelenkes 19 in einer zueinander parallelen Lage, wenn sich die Traganordnung 14 in ihrer Arbeitsstellung befindet. Wegen der beschriebenen Parallelität der Längsrichtungen 54, 55 zu den horizontalen Achsen 5, 20 ist die Lage der horizontalen Achsen 5, 20, zueinander gegenüber auf die Lithotripsieeinrichtung 2 und die Röntgenvorrichtung 3 in horizontaler Richtung wirkenden Kräften gut stabilisiert.

Befindet sich die Traganordnung 14 in Arbeitsstellung und sind die Lithotripsieeinrichtung 2 und die Röntgenvorrichtung 3 aneinander gekoppelt, und sind die Ebenen 26, 27 des Haltearmes 18 und des C-Bogens 6 etwa parallel zueinander, wird mit Hilfe der Laserstrahlen 83, 87 die Lage der Ebenen 26, 27 zueinander bestimmt und gegebenenfalls korrigiert. Dabei werden Bezugsmarkierungen an der Innenseite 84 des C-Bogens 6 verwendet.

## Patentansprüche

1. Modular koppelbare Geräteanordnung (1) mit einer Lithotripsieeinrichtung (2) und einer Röntgenvorrichtung (3), die lösbar miteinander verbindbar in eine vorbestimmte Lage zueinander bringbar sind, wobei die Lithotripsieeinrichtung (2) und die Röntgenvorrichtung (3) jeweils mit einer Kopplungskomponente (31, 32) versehen sind, **dadurch gekennzeichnet, daß** die erste Kopplungskomponente (31) eine Ausnehmung (37) aufweist, welche in ihrer Längsrichtung (55) sich wenigstens abschnittsweise keilartig verjüngend ausgebildet ist, und eine zweite Kopplungskomponente (32) einen in die Ausnehmung (37) wenigstens bereichsweise einführbaren Schuh (45) aufweist, welcher in seiner Einführrichtung (54) wenigstens abschnittsweise keilartig (51) ausgebildet ist, wobei Keilseitenflächen (46, 47) des Schuhs (45) in gekoppeltem Zustand an einem keilartigen Abschnitt der Ausnehmung (37) zentrierend anliegen, wobei
eine Spanneinrichtung (57) vorgesehen ist, mit welcher zwischen der ersten und der zweiten Kopplungskomponente (31, 32) getriebeartig eine Verspannkraft aufbaubar ist, mit welcher die Kopplungskomponenten in eine Kopplungsendlage zueinander gebracht werden.

2. Geräteanordnung (1) nach Anspruch 1,
**dadurch gekennzeichnet,**
**dass** wenigstens eine Keilseitenfläche (46, 47) mindestens eine nach außen seitlich hervorragende Anlagefläche (52, 53) aufweist, gegenüber der sich das Querschnittsprofil des Schuhs (45) etwa quer zu dessen Längsrichtung (54) wenigstens abschnittsweise verjüngt.

3. Geräteanordnung (1) nach einem der Ansprüche 1 oder 2,
**dadurch gekennzeichnet,**
**dass** die Anlagefläche (52, 53) ballig ausgerundet ausgeführt ist.

4. Geräteanordnung (1) nach wenigstens einem der vorhergehenden Ansprüche,
**dadurch gekennzeichnet,**
**dass** der Schuh (45) an einem vorderen Endbereich (49) in Längsrichtung (54) dachartig angeschrägt (50) ausgebildet ist.

5. Geräteanordnung (1) nach wenigstens einem der vorhergehenden Ansprüche,
**dadurch gekennzeichnet,**
**dass** eine der Kopplungskomponenten (31) wenigstens abschnittsweise aus Kunststoff ausgebildet ist, vorzugsweise aus einem PTFE-Werkstoff, und die andere Kopplungskomponente (32) wenigstens abschnittsweise aus einem Metall besteht, vorzugsweise aus Stahl.

6. Geräteanordnung (1) nach wenigstens einem der vorhergehenden Ansprüche,
**dadurch gekennzeichnet,**
**dass** der Schuh (45) in seiner Längsrichtung wenigstens bereichsweise einen sich im Winkel (α) von mindestens etwa 17° verjüngenden Querschnitt (51) hat, vorzugsweise im Winkel (α) von etwa 20°.

7. Geräteanordnung nach wenigstens einem der vorhergehenden Ansprüche,
**dadurch gekennzeichnet,**
**dass** die Kopplungskomponenten (31, 32) jeweils im Bodenbereich (29) der Röntgenvorrichtung (3) und der Lithotripsieeinrichtung (2) vorgesehen sind.

8. Geräteanordnung (1) nach wenigstens einem der vorhergehenden Ansprüche,
**dadurch gekennzeichnet,**
**dass** die Längsrichtung (55) der Ausnehmung (37) und die Längsrichtung (54) des Schuhs (45) etwa parallel sind zu einer Ebene (26) des C-Bogens (6) der Röntgenvorrichtung (3) und zu einer Ebene (27) eines Haltearmes (18) der Lithotripsieeinrichtung (2), wenn sich die Röntgenvorrichtung (3) und die Lithotripsieeinrichtung (2) in gekoppeltem Zustand jeweils in einer Arbeitsstellung befinden.

9. Geräteanordnung (1) nach wenigstens einem der vorhergehenden Ansprüche,
**dadurch gekennzeichnet,**
**dass** an einer der Kopplungskomponenten (31) etwa senkrecht zu deren Längsrichtung (55) ein Haltevorsprung (62) vorgesehen ist, und an der anderen Kopplungskomponente (32) die Spanneinrichtung (57) vorgesehen ist, mit welcher der Haltevorsprung (62) arretierend in Eingriff bringbar ist.

10. Geräteanordnung (1) nach Anspruch 9,
**dadurch gekennzeichnet,**
**dass** die Spanneinrichtung einen Exzentermechanismus (57) mit einer Führungsausnehmung (61) aufweist, in welcher der Haltevorsprung (62) führbar ist.

11. Geräteanordnung (1) nach einem der Ansprüche 9 oder 10,
**dadurch gekennzeichnet,**
**dass** der Haltevorsprung (62) eine Rolle (64) aufweist.

12. Geräteanordnung (1) nach einem der Ansprüche 10 oder 11,
**dadurch gekennzeichnet,**
**dass** die Führungsausnehmung (61) etwa quer zu ihrer Erstreckung eine Rasteinbuchtung (72) aufweist, in welcher der Haltevorsprung (62) in Schließstellung wenigstens abschnittsweise arretierend aufnehmbar ist.

13. Geräteanordnung (1) nach wenigstens einem der Ansprüche 9 bis 12,
**dadurch gekennzeichnet,**
**dass** die Spanneinrichtung (57) eine Rückstellfeder (68) aufweist, welche in Richtung einer Öffnungsstellung der Spanneinrichtung (57) rückstellend wirkt, in welcher der Haltevorsprung (62) in die Führungsausnehmung (61) einführbar ist.

14. Geräteanordnung (1) nach Anspruch 13,
**dadurch gekennzeichnet,**
**dass** die Rückstellfeder (68) in einer Rückstellfederkammer (69) der Spanneinrichtung (57) vorgesehen ist.

15. Geräteanordnung (1) nach wenigstens einem der vorhergehenden Ansprüche,
**dadurch gekennzeichnet,**
**dass** wenigstens eine der Kopplungskomponenten (31) zwischen einer Kopplungsstellung und einer Parkstellung klappbar ist.

16. Geräteanordnung (1) nach Anspruch 15,
**dadurch gekennzeichnet,**
**dass** die Klapprichtung etwa quer zu der Längsrichtung (54, 55) der Ausnehmung (37) und des Schuhs (45) ist, in der gekoppelt wird.

17. Geräteanordnung (1) nach wenigstens einem der vorhergehenden Ansprüche,
**dadurch gekennzeichnet,**
**dass** an einem Haltearm (18) der Lithotripsieeinrichtung (2), welcher in der Ebene (26) des C-Bogens (6) der Röntgenvorrichtung (3) angeordnet ist, zwei Laserpointer (82, 86) vorgesehen sind, die sich in einer Ausrichtung befinden, in der ihre Laserstrahlen (83, 87) etwa in dieser Ebene (26) auf den C-Bogen ausgerichtet verlaufen.

18. Geräteanordnung (1) nach Anspruch 17,
**dadurch gekennzeichnet,**
**dass** ein erster Laserpointer (82) an einem Horizontaldrehgelenkteil (81) des Haltearmes (18) an einer der Abzweigungsstelle des Haltearmes (18) von dem Gelenk (19) etwa gegenüberliegenden Seite des Horizontaldrehgelenkteiles (81) angeordnet ist.

19. Geräteanordnung (1) nach einem der Ansprüche 17 oder 18,
**dadurch gekennzeichnet,**
**dass** ein zweiter Laserpointer (86) an einem therapiekopfseitigen Teil (85) des Haltearmes (18) an einer dem Therapiekopf (13) etwa gegenüberliegenden Seite vorgesehen ist.

20. Geräteanordnung (1) nach Anspruch 19,
**dadurch gekennzeichnet,**
**dass** ein Strahl (87) des zweiten Laserpointers (86) entlang einer schräg im Raum verlaufenden Achse (22) verläuft, um welche der Therapiekopf (13) gegenüber dem ihn tragenden Haltearm (18) schwenkbar ist.

21. Geräteanordnung nach wenigstens einem der Ansprüche 17 bis 20,
**dadurch gekennzeichnet,**
**dass** in einem gekoppelten Zustand der Röntgenvorrichtung (3) und der Lithotripsieeinrichtung (2), in welchem die Ebene (27) des Haltearmes (18) und die Ebene (26) des C-Bogens (6) annähernd zusammenfallen, die beiden Laserpointer (82, 86) die Innenseite (84) einer jeweils anderen Hälfte (7, 9) des C-Bogens (6) anstrahlen.

## Claims

1. Modular couplable apparatus combination (1), comprising a lithotripsy device (2) and an X-ray device (3) that can be brought into a predetermined position with respect to one another in a detachably connectable manner, the lithotripsy device (2) and the X-ray device (3) being provided with a respective coupling component (31, 32), wherein the first coupling component (31) has a recess (37) that has an at least zonally wedge-like tapering configuration in its longitudinal direction (55), and a second coupling component (32) has a shoe (45) which is insertable at least zonally in the recess (37) and which has a wedge-like (51) configuration at least zonally in its insertion direction (54), lateral wedge surfaces (46, 47) of the shoe (45) abutting a wedge-like section of the recess (37) in a centring manner in the coupled state, **characterised in that** a clamping device (57)is provided, by means of which a clamping force can be applied between the first and second coupling component (31,32) in a gear-like manner, by means of which clamping force the coupling components can be brought into a final coupling position with respect to each other.

2. Apparatus combination (1) according to Claim 1, **characterised in that** at least one lateral wedge surface (46, 47) has at least one abutment face (52, 53) projecting laterally outwards, opposite which the cross-sectional profile of the shoe (45) tapers inwards at least zonally approximately transversely to its longitudinal direction (54).

3. Apparatus combination (1) according to either of Claims 1 and 2, **characterised in that** the abutment face (52, 53) has a rounded, crowned configuration.

4. Apparatus combination (1) according to at least one of the preceding claims, **characterised in that** the shoe (45) has a roof-like chamfered (50) configuration in a front end region (49) in the longitudinal direction (54).

5. Apparatus combination (1) according to at least one of the preceding claims, **characterised in that** one of the coupling components (31) is formed at least zonally of plastics material, preferably a PTFE material, and the other coupling component (32) consists at least zonally of a metal, preferably steel.

6. Apparatus combination (1) according to at least one of the preceding claims, **characterised in that** the shoe (45) has a cross section (51) which tapers inwards at least zonally in its longitudinal direction by an angle (α) of at least approximately 17°, preferably an angle (α) of approximately 20°.

7. Apparatus combination according to at least one of the preceding claims, **characterised in that** the coupling components (31, 32) are provided in the base region (29) of the X-ray device (3) and of the lithotripsy device (2) in each case.

8. Apparatus combination (1) according to at least one of the preceding claims, **characterised in that** the longitudinal direction (55) of the recess (37) and the longitudinal direction (54) of the shoe (45) are approximately parallel to a plane (26) of the C-arc (6) of the X-ray device (3) and to a plane (27) of a retaining arm (18) of the lithotripsy device (2), when the X-ray device (3) and the lithotripsy device (2) are each in a working position in the coupled state.

9. Apparatus combination (1) according to at least one of the preceding claims, **characterised in that** a retaining projection (62) is provided on one of the coupling components (31) approximately perpendicularly to the longitudinal direction (55) thereof, and a clamping device (57) is provided on the other coupling component (32), the retaining projection (62) can be brought into locking engagement with the clamping device.

10. Apparatus combination (1) according to Claim 9, **characterised in that** the clamping device has an eccentric mechanism (57) with a guide recess (61) into which the retaining projection (62) can be inserted.

11. Apparatus combination (1) according to either of Claims 9 and 10, **characterised in that** the retaining projection (62) includes a roller (64).

12. Apparatus combination (1) according to either of Claims 10 and 11, **characterised in that** the guide recess (61) has approximately transversely to its extension a latching depression (72) in which the retaining projection (62) can be received at least zonally in a locking manner in the closed position.

13. Apparatus combination (1) according to at least one of Claims 9 to 12, **characterised in that** the clamping device (57) has a return spring (68) which has a restoring action in the direction of an open position of the clamping device (57), in which the retaining projection (62) can be inserted in the guide recess (61).

14. Apparatus combination (1) according to Claim 13, **characterised in that** the return spring (68) is provided in a return spring chamber (69) of the clamping device (57).

15. Apparatus combination (1) according to at least one of the preceding claims, **characterised in that** at least one of the coupling components (31) can be folded between a coupling position and a parked position.

16. Apparatus combination (1) according to Claim 15, **characterised in that** the folding direction is approximately transverse to the longitudinal direction (54, 55) of the recess (37) and the shoe (45), in which coupling takes place.

17. Apparatus combination (1) according to at least one of the preceding claims, **characterised in that** two laser pointers (82, 86) are provided on a retaining arm (18) of the lithotripsy device (2), which retaining arm (18) is arranged in the plane (26) of the C-arc (6) of the X-ray device (3), and which laser pointers (82, 86) are in an orientation in which their laser beams (83, 87) are directed at the C-arc approximately in this plane (26).

18. Apparatus combination (1) according to Claim 17, **characterised in that** a first laser pointer (82) is arranged on a horizontal rotary joint part (81) of the retaining arm (18) on a side of the horizontal rotary joint part (81) located approximately opposite the point where the retaining arm (18) branches from the joint (19).

19. Apparatus combination (1) according to either of Claims 17 and 18, **characterised in that** a second laser pointer (86) is provided on a part (85) of the retaining arm (18) at the therapy head end, on a side located approximately opposite the therapy head (13).

20. Apparatus combination (1) according to Claim 19, **characterised in that** a beam (87) of the second laser pointer (86) is disposed along an axis (22) running obliquely in space, about which the therapy head (13) can be swivelled with respect to the retaining arm (18) supporting it.

21. Apparatus combination according to at least one of Claims 17 to 20, **characterised in that** in a coupled state of the X-ray device (3) and the lithotripsy device (2), in which the plane (27) of the retaining arm (18) and plane (26) of the C-arc (6) approximately coincide, the two laser pointers (82, 86) irradiate the inside (84) of a respective other half (7, 9) of the C-arc (6).

## Revendications

1. Système d'accouplement modulaire (1), comprenant un dispositif de lithotripsie (2) et un dispositif à rayons X (3), susceptibles d'être placés mutuellement en une position prédéterminée en tant susceptibles d'être reliés ensemble de façon désolidarisable, le dispositif de lithotripsie (2) et le un dispositif à rayons X (3) étant chacun muni d'un composant d'accouplement (31, 32), le premier composant d'accouplement (31) présentant un évidement (37) configuré en s'effilant de façon cunéiforme, au moins par tronçons, dans sa direction longitudinale (55), et un deuxième composant d'accouplement (32) présentant un patin (45), susceptible d'être introduit, au moins par zones, dans l'évidement (37), le patin (45) étant réalisé en forme de coin (51), au moins par tronçons, dans sa direction d'insertion (54), les faces latérales de coin (46, 47) du patin (45), à l'état couplé, étant en appui centrant sur un tronçon du genre d'un coin de l'évidement (37),
**caractérisé en ce qu'**
un dispositif de serrage (57) est prévu, à l'aide duquel, entre le premier et le deuxième composant (31, 32), à la façon d'une transmission, est susceptible d'être établie une force de serrage, à l'aide de laquelle les composants d'accouplement sont placés mutuellement en une position finale d'accouplement.

2. Système d'accouplement modulaire (1) selon la revendication 1,
**caractérisé en ce qu'**
au moins une surface latérale de coin (46, 47) présente au moins une surface d'appui (52, 53) ressortant latéralement vers l'extérieur, par rapport à laquelle le profil de section transversale du patin (45) va en s'effilant, au moins par tronçons, à peu près transversalement par rapport à sa direction longitudinale (54).

3. Système d'accouplement modulaire (1) selon la revendication 1 ou 2,
**caractérisé en ce que**
la surface d'appui (52, 53) est arrondi de façon bombée.

4. Système d'accouplement modulaire (1) selon au moins l'une des revendications précédentes,
**caractérisé en ce que**
le patin (45), sur une zone d'extrémité avant (49), est biseauté (50) à la manière d'un toit, en direction longitudinale (54).

5. Système d'accouplement modulaire (1) selon au moins l'une des revendications précédentes, **caractérisé en ce que**
l'un des composants d'accouplement (31) est réalisé, au moins par tronçons, en matière synthétique, de préférence en un matériau de type PTFE, et l'autre composant d'accouplement (32) est composé, au moins par tronçons, d'un métal, de préférence d'acier.

6. Système d'accouplement modulaire (1) selon au moins l'une des revendications précédentes,
**caractérisé en ce que**
le patin (45) présente, dans sa direction longitudinale, au moins par zone, une section transversale (51) allant en s'effilant sous un angle (α) d'au moins à peu près 17°, de préférence sous 20 normes (α) d'à peu près 20°.

7. Système d'accouplement modulaire (1) selon au moins l'une des revendications précédentes,
**caractérisé en ce que**
les composants d'accouplement (31, 32) sont chacun prévus dans la zone de fond (29) du dispositif à rayons X (3) et du dispositif de lithotrypsie (2).

8. Système d'accouplement modulaire (1) selon au moins l'une des revendications précédentes, **caractérisé en ce que**
la direction longitudinale (55) de l'évidement (37), et la direction longitudinale (54) du patin (45), sont à peu près parallèles à un plan (26) de l'arc en C (6) du dispositif à rayons X (3) et à un plan (27) d'un bras de maintien (18) du dispositif de lithotripsie (2), lorsque le distinctif à rayons X (3) est le dispositif de lithotripsie (2), à l'état couplés, se trouvent chacun en une position de travail.

9. Système d'accouplement modulaire (1) selon au moins l'une des revendications précédentes,
**caractérisé en ce**,
sur l'un des composants d'accouplement (31) est prévue, à peu près perpendiculairement à sa direction longitudinale (55), une série de maintien (62) et, sur l'autre composant d'accouplement (32), est prévu le dispositif de serrage (57), à l'aide duquel la saillie de maintien (62) est susceptible d'être mise en prise avec effet de blocage.

10. Système d'accouplement modulaire (1) selon la revendication 9,
**caractérisé en ce que**
le dispositif de serrage présent un mécanisme à excentrique (57) avec un évidement de guidage (61), dans lequel la saillie de maintien (62) est susceptible d'être guidée.

11. Système d'accouplement modulaire (1) selon la revendication 9 ou 10,
**caractérisé en ce que**
la saillie de maintien (62) présente un galet (64).

12. Système d'accouplement modulaire (1) selon l'une des revendications 10 ou 11,
**caractérisé en ce que**
l'évidement de guidage (61) présente, à peu près transversalement à son étendue, une bosse rentrante d'encliquetage (72), dans laquelle la saillie de maintien (62), en position de fermeture, peut être logée, au moins par tronçons, avec effet de blocage.

13. Système d'accouplement modulaire (1) selon l'une des revendications 9 à 12,
**caractérisé en ce que**
le dispositif de serrage (57) présente un ressort de rappel (68), agissant avec effet de rappel dans le sens d'une position d'ouverture du dispositif de serrage (57), dans laquelle la saillie de maintien (62) peut être insérée dans l'évidement de guidage (61).

14. Système d'accouplement modulaire (1) selon la revendication 13,
**caractérisé en ce que**
le ressort de rappel (68) est prévu dans une chambre à ressort de rappel (69) du dispositif de serrage (57).

15. Système d'accouplement modulaire (1) selon au moins l'une des revendications précédentes,
**caractérisé en ce qu'**
au moins l'un des composants d'accouplement (31) est susceptible d'être rabattu entre une position d'accouplement et une position de rangement.

16. Système d'accouplement modulaire (1) selon la revendication 15,
**caractérisé en ce que**
le sens de rabattement est à peu près transversal à la direction longitudinale (54, 55) de l'évidement (37) et du patin (45) dans laquelle il est couplé.

17. Système d'accouplement modulaire (1) selon au moins l'une des revendications précédentes,
**caractérisé en ce que,**
sur un bras de maintien (18) du dispositif de lithotrispie (2), disposés dans le plan (26) de l'arc en C (6) du dispositif à rayons X (3), sont prévues deux pointeurs à laser (82, 86), se trouvant sous les orientations dans laquelle leurs rayons laser (83, 87) sont orientés à peu près dans ce plan (26) sur l'arc en C.

18. Système d'accouplement modulaire (1) selon la revendication 17,
**caractérisé en ce qu'**
un premier pointeur laser (82) est disposé sur une partie d'articulation tournante horizontale (81) du bras de maintien (18), sur un côté, à peu près opposé à l'emplacement de ramifications du bras de maintien (18) vis-à-vis de l'articulation (19), de la partie d'articulation horizontale (81).

19. Système d'accouplement modulaire (1) selon l'une des revendications 17 ou 18,
**caractérisé en ce qu'**
un deuxième pointeur à laser (86) est prévu sur une partie (85), située côté tête de thérapie, du bras de maintien (18), sur un côté à peu près opposé à la tête de thérapie (13).

20. Système d'accouplement modulaire (1) selon la revendication 19,
**caractérisé en ce qu'**
un rayon (87) du deuxième pointeur laser (86) s'étend le long d'un axe (22) s'étendant obliquement dans l'espace, autour duquel la tête de thérapie (13) est susceptible de pivoter par rapport au bras de maintien (18) le portant.

21. Système d'accouplement modulaire (1) selon l'une des revendications 17 à 20,
**caractérisé en ce que,**
en un état couplé du dispositif à rayons X (3) et du dispositif de lithotripsie (2), dans lequel le plan (27) du bras de maintien (18) et le plan (26) de l'arc en C (6) coïncident à peu près, les doux pointeurs à laser (82, 86) émettent un rayonnement sur la face intérieure (84) de chaque fois l'autre moitié (7, 9) de l'arc en C (6).
